# EUROPÄISCHE PATENTSCHRIFT

(11) **EP 0 822 842 B1**
(45) Veröffentlichungstag und Bekanntmachung des Hinweises auf die Patenterteilung: **28.08.2002**
(21) Anmeldenummer: 96909067.9
(22) Anmeldetag: 13.04.1996
(51) Int. Cl.: A61M 16/04

(54) **TRACHEOSTOMIEKANÜLE**
TRACHEOSTOMY CANNULA
CANULE POUR TRACHEOSTOMIE

(30) Priorität: 24.04.1995 DE 19514433
(43) Veröffentlichungstag der Anmeldung: 11.02.1998
(73) Patentinhaber: TRACOE medical GmbH, 60528 Frankfurt am Main (DE)
(72) Erfinder: WALDECK, Franz, D-55268 Nieder-Olm (DE)
(74) Vertreter: Weber, Dieter, Dr.
(86) Internationale Anmeldenummer: DE9600688
(87) Internationale Veröffentlichungsnummer: WO96033760

(56) Entgegenhaltungen:
- EP-A- 0 598 948
- GB-A- 2 007 789
- GB-A- 2 205 504
- US-A- 3 659 612
- US-A- 4 033 353
- US-A- 5 361 754

## Beschreibung

Die Erfindung betrifft eine Tracheostomiekanüle zum Einsatz in ein Tracheostoma nach dem Oberbegriff des Anspruches 1. Sie weist eine schlauchförmige Außenkanüle, in welche eine ebenfalls schlauchförmige Innenkanüle führbar und mit der Außenkanüle am proximalen Teil verriegelbar ist, auf , wobei im proximalen Bereich der Außenkanüle ein Kanülenschild zur Anlage am Hals des Patienten angebracht ist. Außenkanüle und Innenkanüle zusammen bilden das Kanülenrohr.

Derartige Tracheostomiekanülen mit nur um eine Achse verschwenkbarem Kanülenschild sind seit längerem bekannt. Aus der Patentliteratur sei hier auf die US-PS 5,067,496, EP-0 107 779 B1,EP-0 037 719 B1, US-PS 4,852,565 und US-PS 4,009,720 hingewiesen.

Derartige Tracheostomiekanülen werden in ein operativ hergestelltes Tracheostoma eingeführt, um es, solange es heilt, offen zu halten. Es gibt aber auch Fälle, in denen eine Tracheostomiekanüle permanent im Tracheostoma verbleiben muß. In weiteren Fällen dient die Tracheostomiekanüle der künstlichen Beatmung.

Mit Hilfe eines sogenannten Führungsstabes (Mandrin bzw. Obturator) kann die Außenkanüle unter Aufweitung des Tracheostomas in die Trachea eingeführt werden. Die Außenkanüle hält das Tracheostoma offen und braucht nur ab und zu zum Säubern entfernt zu werden. Proximal an der Außenkanüle ist ein sogenanntes Kanülenschild angebracht, welches sich gegen den Hals des Patienten anlegt, wenn die Tracheostomiekanüle in dem Tracheostoma sitzt. Das Kanülenschild verhindert unter anderem, daß die Tracheostomiekanüle zu weit in die Trachea hinein geführt wird oder gar in diese hineinrutscht.

In die Außenkanüle paßt exakt die entsprechend ausgebildete schlauchförmige Innenkanüle, die aber daraus wieder leicht entnehmbar ist, um nämlich häufig gereinigt zu werden. Dies hat den Vorteil, daß die Außenkanüle dazu nicht aus der Trachea und dem Tracheostoma herausgenommen werden muß, was anderenfalls zu Schmerzen und Verletzungen führen könnte.

Die aus den oben zitierten Druckschriften bekannten Tracheostomiekanülen weisen ein Kanülenschild auf, gegenüber dem das Kanülenrohr schwenkbar um eine Achse, nämlich um die waagerechte Achse, gelagert ist. Das Kanülenschild weist hierzu eine Durchbrechung auf, durch welche hindurch die Außenkanüle greift. In die Durchbrechung hinein ragen zwei Zapfen, die in entsprechenden Aufnahmen im Außenbereich der Außenkanüle greifen, derart, daß das Kanülenrohr in einem gewissen Bereich von ca. ± 45° gegenüber dem Kanülenschild schwenkbar ist. Durch diese Schwenkbarkeit wird ein gewisser Tragekomfort erzielt, insofern, als sich die Tracheostomiekanüle beim Beugen oder Recken des Kopfes des Patienten in ihrer Lage etwas anpassen kann, so daß die Schmerzen, die ein Patient empfindet, wenn die Kanüle beispielsweise an die vordere oder hintere Tracheawand gedrückt wird, vermindert werden.

Allerdings ist das Ergebnis des Tragekomforts der bekannten Tracheostomiekanülen noch nicht befriedigend.

Eine gattungsgemäße Tracheostomiekanüle ist bekannt geworden aus der US-A-4.686.977. Darin beschrieben ist eine Tracheostomiekanüle, bei der das Kanülenschild durch ein Kugelgelenk im proximalen Teil der Kanüle gelagert ist. Diese Art der Lagerung erlaubt das Verschwenken des Kanülenrohres gegenüber dem Kanülenschild um die drei Raumachsen X, Y und Z. Mit diesem Vorschlag wurde ein theoretischer Ansatz dafür geschaffen, wie der Tragekomfort einer solchen Tracheostomiekanüle verbessert werden könnte. Die Realisierung dieser Tracheostomiekanüle stößt jedoch auf Schwierigkeiten. So bietet sie zum einen keine ausreichende Verschwenkbarkeit des Kanülenrohres gegenüber dem Kanülenschild, da die äußeren halbschaligen Backen im Interesse der Sicherheit die Kugel des Gelenks vergleichsweise weit umfassen müssen, da ansonsten das Risiko besteht, daß die Kanüle sich aus der Lagerung löst und in die Trachea fällt. Zum anderen ist die Befestigung der Innenkanüle schwierig herzustellen, insbesondere im Hinblick auf das Auswechseln der Innenkanüle, was recht häufig vorgenommen werden muß. Für die Befestigung der Innenkanüle bietet die genannte Druckschrift keinerlei Lösungsansätze. Es wären daher die bekannten Befestigungsarten denkbar, wie beispielsweise durch einen Bayonettverschluß. Allerdings würde bei der Herstellung der Verbindung mit einem Bayonettverschluß eine Bewegung um die dritte Raumachse Z stattfinden. Diese Drehung ist jedoch für den Patienten sowohl im Wachzustand als auch im beispielsweise Narkosezustand gefährlich, da sich ansonsten ohne weitere Maßnahme das Kanülenrohr seitlich an die Tracheawand zu stark andrückt.

Vor diesem Hintergrund ist es nun die Aufgabe der vorliegenden Erfindung, eine gattungsgemäße Tracheostomiekanüle so weiterzubilden, daß die Verschwenkbarkeit um zwei Raumachsen im ausreichenden Maße möglich ist und die Verschwenkbarkeit auf eine relativ einfache technische Art realisiert wird.

Gelöst wird diese Aufgabe erfindungsgemäß durch die Tracheostomiekanüle mit den Merkmalen des kennzeichnenden Teils des Anspruchs 1. Weitere vorteilhafte Ausführungsformen ergeben sich aus den Unteransprüchen.

Demnach ist vorgesehen, daß die Verschwenkbarkeit um die Y-Raumachse durch einen um die Außenkanüle greifenden und an dieser drehgelagerten Ring und die Verschwenkbarkeit um die X-Achse durch die Drehlagerung des Kanülenschildes an dem Ring erzielt wird. Betrachtet man das Kanülenschild idealisiert als Ring, so ist die Erfindung dadurch realisiert, daß zwei gegeneinander verdrehbare Ringe, nämlich der Innenring gegenüber dem Außenring (Kanülenschild) verschwenkbar an der Außenkanüle gelagert sind. Diese Realisierung ist konstruktiv relativ einfach, aber sehr wirksam und kostengünstig.

Die Drehlagerung im vorerwähnten Falle des Ringes an der Außenkanüle und des Kanülenschildes an dem Ring sind vorzugsweise realisiert durch Zapfen, die in entsprechend ausgebildete Aufnahmen an der Kanüle, dem Ring und dem Kanülenschild ausgebildet sein können. Dies hängt davon ab, wo die Zapfen angeordnet sind.

Die Dimensionen im vorerwähnten Falle sind vorzugsweise so gewählt, daß das Kanülenrohr gegenüber dem Kanülenschild um die Y-Achse um ± 25° und um die X-Achse um ± 60° verschwenkbar ist.

Die Erfindung wird nachfolgend anhand eines Ausführungsbeispiels gemäß der Zeichnungsfiguren näher erläutert. Hierbei zeigt:
Fig. 1 die Tracheostomiekanüle in Ansicht mit Cuff und Kontrollballon.
Fig. 2 eine Schnittansicht durch die Tracheostomiekanüle gemäß Fig. 1,
Fig. 3 eine Detailansicht in Richtung des Pfeiles III in Fig. 1,
Fig. 4 eine ähnliche Ansicht wie in Fig. 3, jedoch mit nach einer Seite verschwenktem kanülenschild,
Fig. 5 eine ähnliche Ansicht wie Fig. 4 mit dem zur anderen Seite verschwenkten Kanülenschild.
Fig. 6 eine Detailansicht des proximalen Bereichs der Tracheostomiekanüle gemäß Fig. 1 mit um die X-Achse in einer Richtung verschwenktem Kanülenschild, und
Fig. 7 eine ähnliche Ansicht wie Fig. 6 mit zu der anderen Seite verschwenktem Kanülenschild.

Nachfolgend sind die gleichen Teile mit denselben Bezugszeichen versehen.

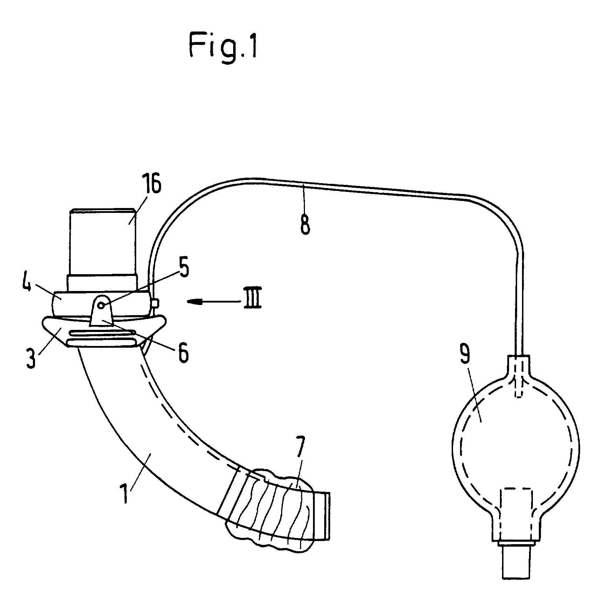

Fig. 1 dient dem ersten groben Überblick über die erfindungsgemäße Tracheostomiekanüle. Diese besteht aus einer Außenkanüle 1, in welche eine Innenkanüle (in Fig. 1 nicht erkennbar) geführt ist. An der Innenkanüle ist ein Anschlußteil 16 nach DIN ISO 5356-1 (1987) angeformt, welches aus dem Tracheostoma des Patienten herausragt. Wenn die Tracheostomiekanüle vollständig durch das Tracheostoma in die Trachea eingeführt worden ist, legt sich das Kanülenschild 3, welches im proximalen Bereich der Außenkanüle 1 angebracht ist, gegen den Patientenhals.

Das aus der Außenkanüle und der Innenkanüle bestehende Kanülenrohr ist durch die mit dem Bezugszeichen 4, 5 und 6 bezeichneten Teile um zwei Raumachsen verschwenkbar gegenüber dem Kanülenschild (3) angeordnet, wie weiter unten noch eingehender erläutert werden wird.

Vorliegend ist im distalen Bereich der Außenkanüle 1 ein Ballon oder Cuff 7 angeordnet. welcher über eine Luftleitung 8 von extrakorporal her aufgepumpt werden kann, damit das distale Ende der Tracheostomiekanüle in eine definierte Stellung zur Tracheawand gebracht werden kann und damit eine Abdichtung zwischen Außenkanüle und Luftröhre erreicht werden kann. Dies wird mittels des Aufblasens des Ballons oder Cuffs 7 von außen erreicht und durch den Kontrollballon 9 kontrolliert.

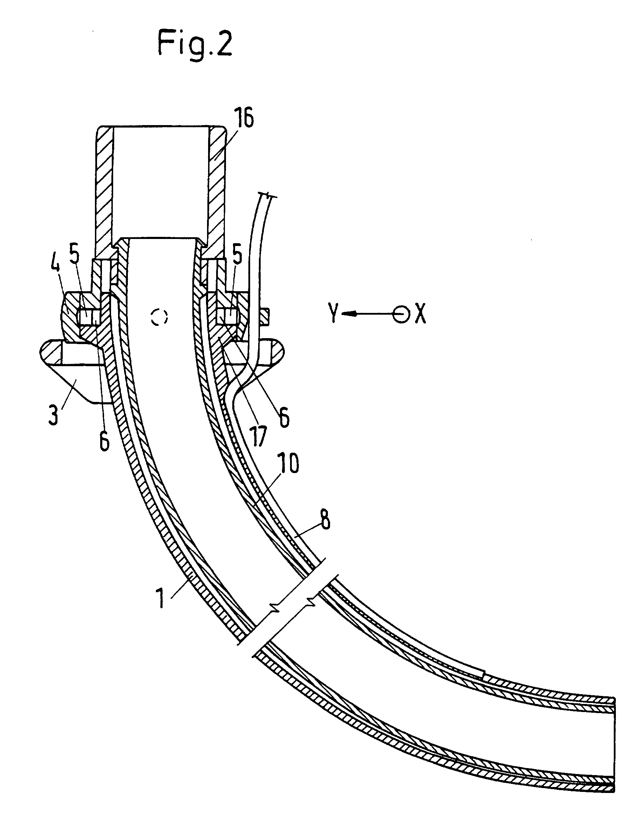

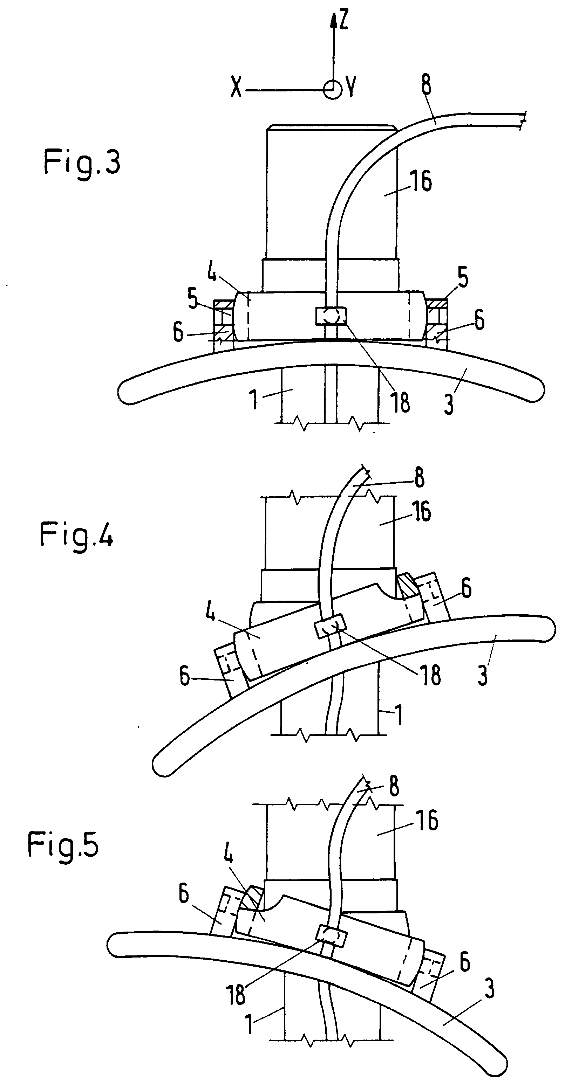

Einzelheiten des konstruktiven Aufbaus der Tracheostomiekanüle lassen sich der Figur 2 entnehmen, welche eine Schnittansicht zeigt. Klar erkennbar ist hier, wie die Innenkanüle 10 in die Außenkanüle 1 hinein geführt ist. Die Verschwenkbarkeit des Kanülenschildes 3 um die in der Zeichnungsfigur waagerechte Achse (Y-Achse) wird vorliegend dadurch erreicht, daß ein Ring 4 um die Außenkanüle 1 im proximalen Bereich greift und an dieser drehgelagert ist durch in das Ringinnere ragende Zapfen 5, die in Aufnahmen oder Ausnehmungen 6 in einer am proximalen Ende der Außenkanüle 1 angeformten Schulter 17 greifen. Um die Verschwenkbarkeit um die senkrecht zur Zeichnungsebene stehende Achse (X-Achse) zu ermöglichen, ist das Kanülenschild 3 an dem Ring 4 drehgelagert angeschlagen. Dies wird allerdings deutlicher aus den Figuren 3 bis 5.

Die Figur 3 zeigt die vergrößerte Teilansicht in Richtung des Pfeiles III in Fig. 1 auf den proximalen Bereich der Tracheostomiekanüle. Oben ist das Koordinatensystem angegeben, auf welches sich Angaben der Raumachsen beziehen.

Aus Fig. 3 ist zunächst die Drehlagerung des Kanülenschildes 3 an dem Ring 4 zu ersehen. Vom Ring ragen radial zwei Zapfen 5 ab, die in zwei Aufnahmen oder Ösen 6. die am Kanülenschild 3 angeformt sind, greifen. Hierdurch wird die Verschwenkbarkeit um die X-Achse in dem skizzierten Koordinatensystem erreicht.

Erkennbar in Fig. 3 ist wiederum das Anschlußteil 16 sowie die Außenkanüle 1. Auch der Schlauch 8, welcher zum Ballon 7 (Fig. 1) führt, ist erkennbar, und zwar wie er durch und in einer Halteöse 18 auf dem Ring 4 fixiert ist.

Die Figuren 4 und 5 zeigen in sehr anschaulicher Weise die Möglichkeiten der Verschwenkung um die Y-Achse, welche durch die Lagerung, die anhand von Fig. 2 erläutert worden ist, ermöglicht wird.

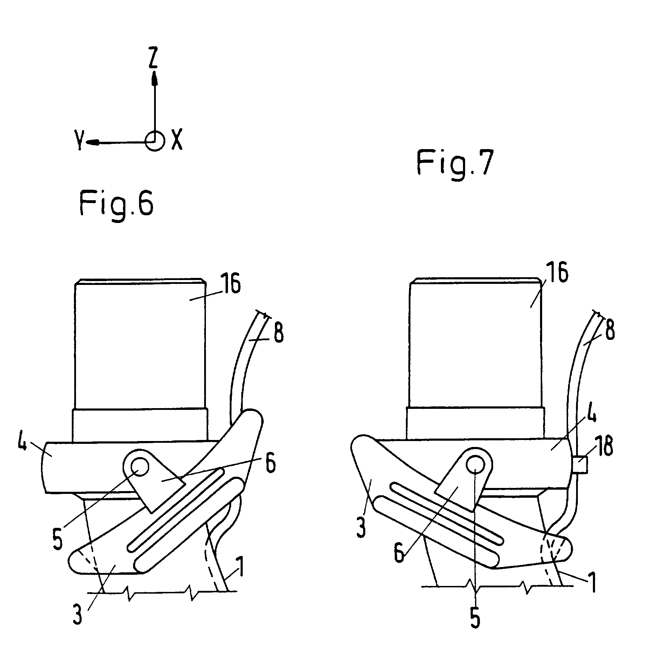

Die Figuren 6 und 7 zeigen die Ansicht des proximalen Bereiches der Tracheostomiekanüle gegenüber den Figuren 3 bis 5 um 90° versetzt, also von rechts gesehen. Hier wird die Verschwenkbarkeit des Kanülenschildes 3 um die X-Achse in anschaulicher Weise dargestellt. Die Einzelheiten der Lagerung ist anhand der Fig. 3 erläutert worden.

## Patentansprüche

1. Tracheostomiekanüle zum Einsatz in ein Tracheostoma, aufweisend eine schlauchförmige Außenkanüle (1), in welche eine ebenfalls schlauchförmige Innenkanüle (10) führbar und mit der Außenkanüle (1) am proximalen Teil verriegelbar ist, um ein Kanülenrohr zu bilden, wobei im proximalen Bereich der Außenkanüle ein Kanülenschild (3) zur Anlage am Hals angebracht ist, durch welches hindurch der proximale Teil der Außenkanüle (1) greift und wobei das Kanülenrohr gegenüber dem Kanülenschild (3) um zwei Raumachsen (X, Y) verschwenkbar gelagert ist, **dadurch gekennzeichnet, daß** die Verschwenkbarkeit um die Y-Raumachse erzielt wird durch einen um die Außenkanüle (1) greifenden und an dieser drehgelagerten Ring (4) und die Verschwenkbarkeit um die X-Achse durch die Drehlagerung des Kanülenschildes (3) an dem Ring (4).

2. Tracheostomiekanüle nach Anspruch 1, **dadurch gekennzeichnet, daß** der Verschwenkbereich um die Y-Achse ± 25° und um die X-Achse ± 60° beträgt.

3. Tracheostomiekanüle nach Anspruch 1 oder 2, **dadurch gekennzeichnet, daß** die Drehlagerung des Ringes (4) an der Außenkanüle (1) und des Kanülenschildes (3) an dem Ring (4) realisiert sind durch Zapfen (5), die in entsprechend ausgebildete Aufnahmen (6) greifen.

## Claims

1. Tracheostomy cannula for use in a tracheostoma provided with a hose-like outer cannula (1) into which an inner cannula (10), which is also hose-like, can be guided and can be locked to the outer cannula (1) at the proximal part in order to form a cannula tube, wherein a cannula plate (3) for application to the neck is fitted in the proximal area of the outer cannula, through which the proximal part of the outer cannula reaches (1) and wherein the cannula tube is mounted such that it is able pivot about two spatial axes (X, Y) with respect to the cannula plate (3), **characterised in that** the ability to pivot about the Y axis is obtained by means of a ring (4) gripping around the outer cannula (1) and rotatably mounted on it, and the ability to pivot about the X axis is obtained by the rotatable mounting of the cannula plate (3) on the ring (4).

2. Tracheostomy cannula according to claim 1, **characterised in that** the range of pivoting about the Y axis is ± 25° and about the X axis is ± 60°.

3. Tracheostomy cannula according to claim 1 or 2, **characterised in that** the rotatable mounting of the ring (4) on the outer cannula (1) and of the cannula plate (3) on the ring (4) is effected by pegs (5) which engage with correspondingly configured cut-outs (6).

## Revendications

1. Canule de trachéostomie destinée à être utilisée dans un trachéostome, comportant une canule extérieure (1) en tuyau souple dans laquelle une canule intérieure (10), également en tuyau souple, peut être insérée et être verrouillée à la canule extérieure (1) au niveau de la partie proximale pour former un tube de canules, une plaque de canule (3) étant placée dans la région proximale de la canule extérieure en vue de la mise en place au niveau du cou en bloquant la partie proximale de la canule extérieure (1) qui la traverse et le tube de canules étant monté de façon à pouvoir pivoter par rapport à la plaque de canule (3) sur deux axes (X, Y) de l'espace, **caractérisée en ce que** le caractère pivotant autour de l'axe Y de l'espace est obtenu par une bague (4) bloquant la canule extérieure (1) sur laquelle elle est montée à rotation et le caractère pivotant autour de l'axe X est obtenu par le montage à rotation de la plaque de canule (3) sur la bague (4).

2. Canule de trachéostomie selon la revendication 1, **caractérisée en ce que** la zone de pivotement autour de l'axe Y est de ±25° et celle autour de l'axe X est de ±60°.

3. Canule de trachéostomie selon la revendication 1 ou 2, **caractérisée en ce que** le montage à rotation de la bague (4) sur la canule extérieure (1) et celui de la plaque de canule (3) sur la bague (4) sont réalisés par des pivots (5) qui s'engagent dans des logements (6) conformés en correspondance.
